(19) Europäisches Patentamt
European Patent Office
Office européen des brevets

(11) Veröffentlichungsnummer : **0 306 451 B1**

(12) **EUROPÄISCHE PATENTSCHRIFT**

(45) Veröffentlichungstag der Patentschrift : **11.12.91 Patentblatt 91/50**

(51) Int. Cl.[5] : **C07C 279/28,** C08G 59/40

(21) Anmeldenummer : **88810581.4**

(22) Anmeldetag : **24.08.88**

(54) **Oligomere Cyanoguanidine.**

(30) Priorität : **02.09.87 CH 3358/87**

(43) Veröffentlichungstag der Anmeldung : **08.03.89 Patentblatt 89/10**

(45) Bekanntmachung des Hinweises auf die Patenterteilung : **11.12.91 Patentblatt 91/50**

(84) Benannte Vertragsstaaten : **AT BE CH DE ES FR GB IT LI NL SE**

(56) Entgegenhaltungen :
**EP-A- 0 126 567**
**DE-A- 2 733 952**

(73) Patentinhaber : **CIBA-GEIGY AG**
**Klybeckstrasse 141**
**CH-4002 Basel (CH)**

(72) Erfinder : **Flury, Peter, Dr.**
**Muldenweg**
**CH-4204 Himmelried (CH)**
Erfinder : **Roth, Martin, Dr.**
**Oberdorf**
**CH-1735 Giffers (CH)**
Erfinder : **Eldin, Sameer H., Dr.**
**Route de Schiffenen 10**
**CH-1700 Fribourg (CH)**

**EP 0 306 451 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft oligomere Cyanoguanidine, ein Verfahren zu deren Herstellung und deren Verwendung als latente Härter für Epoxidharze.

Dicyandiamid hat sich als latenter Härter für Epoxidharze seit langem bewährt (vgl. z.B. H. Lee und K. Neville "Handbook of Epoxy Resins", McGraw Hill, New York, 1982, Seite 10-16) und wird in der Praxis vor allem als Härter für feste Laminierharze verwendet. Dicyandiamid hat aber den gravierenden Nachteil, dass es nur in für das Laminiergeschäft ungeeigneten Lösungsmitteln, wie Wasser, Aceton-Wasser, Methanol, N-Methylpyrrolidon, Dimethylformamid, Hydroxylgruppen enthaltenden Ethern usw. löslich ist. Das heute üblicherweise verwendete Lösungsmittel, 2-Methoxyethanol, ist aus toxikologischen Gründen bedenklich. Zudem weisen mit Dicyandiamid gehärtete Epoxidharze vergleichsweise tiefe Glasumwandlungstemperaturen auf.

Ausserdem sind die zwei folgenden Verbindungen 1,6-Hexan-bis(3-cyano-2-isobutylguanidin) und 1,8-Bis-[N'-(3-(2-thiazolyl)-propyl)-N"-cyanoguanidino]-octan in EP-A 126567 (Seite 28) bzw. in DE-A 2733952 (Beispiel 1b) beschrieben.

Die erfindungsgemässen oligomeren Cyanoguanidine sind, wie Dicyandiamid, latente Härter, welche bei Raumtemperatur stabil sind, während sie bei erhöhter Temperatur eine schnelle Vernetzung der Harze verursachen. Sie sind in für die Applikation von Epoxidharzen geeigneten, unproblematischen Lösungsmitteln gut löslich. Die damit gehärteten vernetzten Epoxidharze zeichnen sich zudem durch eine wesentlich höhere Glasumwandlungstemperatur als entsprechende mit Dicyandiamid gehärtete Systeme aus.

Gegenstand vorliegender Erfindung sind oligomere Cyanoguanidine der Formel I

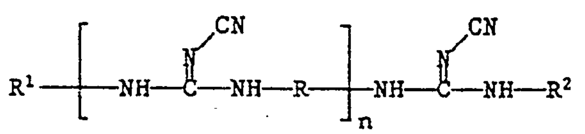

(I),

worin R ein zweiwertiger $C_2$-$C_{20}$-aliphatischer, ein- oder mehrkerniger $C_5$-$C_{20}$-cycloaliphatischer, $C_6$-$C_{20}$-aromatischer oder $C_4$-$C_{20}$-heterocyclischer Rest ist oder für eine Gruppe der Formel II steht

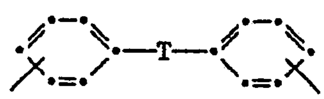

(II),

worin T Methylen, Isopropyliden, CO, O, S oder $SO_2$ bedeutet, $R^1$ und $R^2$ unabhängig voneinander einen $C_1$-$C_{12}$-Alkyl-, $C_5$-$C_{10}$-Cycloalkyl-, $C_6$-$C_{10}$-Aryl-, $C_7$-$C_{12}$-Aralkyl- oder einen $C_3$-$C_8$-heterocyclischen Rest bedeuten und n eine ganze Zahl von 1-20 ist, wobei die Reste R, $R^1$ und $R^2$ unsubstituiert sind oder $C_1$-$C_4$-Alkyl-, $C_1$-$C_4$-Alkoxy-, Nitro-, Halogen-,

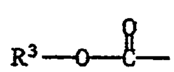 oder 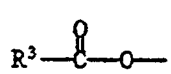

Substituenten enthalten und $R^3$ Phenyl oder $C_1$-$C_4$-Alkyl bedeutet, wobei die zwei folgenden Verbindungen 1,6-Hexan-bis(3-cyano-2-isobutylguanidin) und 1,8-Bis-[N'-(3-(2-thiazolyl)-propyl)-N"-cyanoguanidino]-octan ausgenommen sind.

Die Struktur der erfindungsgemässen Cyanoguanidine der Formel I wurde der Einfachheit halber als 2-Cyanoguanidin (d.h. mit der Cyanogruppe am =N-Stickstoffatom gebunden) dargestellt. Es versteht sich von selber, dass diese Verbindungen auch als tautomere Formen, d.h. als 1- bzw. 3-Cyanoguanidine, vorliegen können und dass die Lage des Gleichgewichts zwischen den möglichen Tautomeren von den Resten $R^1$, $R^2$ und R abhängt.

Monomere Cyanoguanidine des Typs

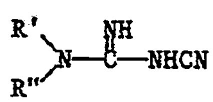

sind als Härter für Acetalharze oder für Polyurethanharze bekannt, wie z.B. in der JP-OS 85/44543 bzw. in der US 3,864,313 beschrieben. Die in der erwähnten JP-OS beschriebenen härtbaren Acetalharz-Stoffgemische enthalten zur Verbesserung der Haftung des Polyacetalharzes und der darin enthaltenen anorganischen Füllstoffe noch kleine Mengen eines Polyglycidylethers oder eines Diglycidylesters.

Die JP-OS 86/207425 offenbart die Verwendung von Gemischen aus monomeren Cyanoguanidinen, insbesondere Dicyandiamid, Polyether-Polyaminen und substituierten Guanidinen als Härter für spezielle Epoxidharze. Diese Härtergemische eignen sich nicht als latente Härter und die damit gehärteten Epoxidharze weisen zudem relativ tiefe Glasumwandlungstemperaturen auf.

Die erfindungsgemässen Verbindungen können durch Erhitzen eines Gemisches enthaltend ein Monoisocyanat $R^1$-NCO und $R^2$-NCO und ein Diisocyanat, OCN-R-NCO in Gegenwart eine Katalysators zu einem oligomeren Carbodiimid der Formel III

$$R^1-\left[-N{=}C{=}N-R-\right]_n-N{=}C{=}N-R^2 \qquad \text{(III)},$$

und anschliessende Umsetzung des Carbodiimids der Formel III mit Cyanamid hergestellt werden, wobei die Symbole $R^1$, $R^2$, R und n die oben angegebene Bedeutung haben. Das erwähnte Herstellungsverfahren ist ein weiterer Gegenstand der vorliegenden Erfindung.

Die Ausgangsverbindungen, die zu verwendenden Mono- und Diisocyanate sind bekannt und können auf bekannte Weise hergestellt werden.

Die katalytische Umsetzung von Isocyanaten zu Carbodiimiden ist ebenfalls bekannt und ist zum Beispiel von S.R. Sandler und W. Karo in "Organic Functional Group Preparation", Band 2 (Organic Chemistry Series Band 12-2), Academic Press, Orlando, FL, U.S.A. 1986, Seiten 233-258, beschrieben. Geeignete Katalysatoren sind z.B. Metallalkoholate und -phenolate, wie Kalium-tert.-butylat, Natriumphenolat oder Titan-iso-propylat, Metallcarbonyle, wie z.B. $Fe(CO)_5$, $W(CO)_6$ oder $Mo(CO)_6$ und insbesondere bestimmte Phosphorverbindungen, wie z.B. Phosphoniumsalze oder Phosphinoxide. Besonders geeignete Katalysatoren sind fünfgliedrige cyclische Phosphinoxide, wie z.B. 1-Ethyl-3-methyl-1-phospha-3-cyclopenten-1-oxid und insbesondere 3-Methyl-1-phenyl-1-phospha-3-cyclopenten-1-oxid. Die Menge des Katalysators beträgt vorzugsweise etwa 0,1-2, insbesondere 0,3-1 Mol% bezogen auf die Edukte.

Die Umsetzung erfolgt geeigneterweise in einem inerten Lösungsmittel, wie z.B. Toluol, Xylol, Cyclohexan, $CCl_4$ usw., bei erhöhter Temperatur, wie z.B. bei über 30°C, vorzugsweise 40-150°C, beispielsweise unter Rückfluss.

Je nach dem Mengenverhältnis des eingesetzten Monoisocyanats zum Diisocyanat entstehen oligomere Carbodiimide der Formel III mit unterschiedlicher Molekulargewichtsverteilung. Im allgemeinen entsteht bei der Umsetzung ein Oligomerengemisch, dessen durchschnittliches Molekulargewicht durch Einsatz von mehr oder weniger Diisocyanat zum Monoisocyant nach Wunsch gesteuert werden kann. Es können aber auch gezielt Produkte mit einem im wesentlichen einheitlichen Molekulargewicht hergestellt werden. Als besonders geeignet hat sich der Einsatz von etwa 0,5 bis 10, vorzugsweise etwa 1 bis 6 Mol Monoisocyanat pro Mol Diisocyanat erwiesen.

Die zweite Stufe der Synthese, die Addition von Cyanamid an das oligomere Carbodiimid erfolgt in der Regel ohne Aenderung des Polymerisationsgrades. Die Umsetzung wird vorzugsweise in Anwesenheit eines basischen Katalysators, z.B. eines tertiären Amins, wie Triethylamin, in einem inerten Lösungsmittel, wie z.B. Diethylether, Tetrahydrofuran, Dioxan oder insbesondere in protischen Lösungsmitteln, wie z.B. Isopropanol, gegebenenfalls bei erhöhter Temperatur, durchgeführt.

Bevorzugt werden oligomere Cyanoguanidine der Formel I, worin n eine ganze Zahl von 1-10, insbesondere von 1-5 ist.

Gegenstand der Erfindung sind auch Cyanoguanidingemische erhältlich durch Erhitzen eines Gemisches enthaltend ein Monoisocyanat $R^1$-NCO und $R^2$-NCO und ein Diisocyanat, OCN-R-NCO in Gegenwart eine Katalysators zu einem Carbodiimid der Formel III*

$$R^1-\left[-N{=}C{=}N-R-\right]_{n^*}-N{=}C{=}N-R^2 \qquad \text{(III*)}$$

und anschliessende Umsetzung des Carbodiimids der Formel III* mit Cyanamid, wobei die Symbole $R^1$, $R^2$ und R die oben angegebene Bedeutung haben und n* Null oder eine ganze Zahl von 1-20 ist.

Der Rest R der Verbindungen der Formel I kann ein zweiwertiger geradkettiger oder verzweigter aliphatischer Rest mit 2-20, vorzugsweise 2-10 und insbesondere 2-6 C-Atomen sein. Beispiele für geeignete aliphatische Reste R sind Ethylen, 1,2- und 1,3-Propylen, Butylen, Penta- und Hexamethylen, Heptylen, Octylen, Decylen, Dodecylen, Hexadecylen und Neopentylen.

R kann auch ein ein- oder mehrkerniger cycloaliphatischer zweiwertiger Rest mit 5-20 C-Atomen sein, wie beispielsweise Cyclopentylen, Cyclohexylen, Cycloheptylen, Cyclooctylen, Bis(cyclohexylen)methan, 2,2-Bis(cyclohexylen)propan, Decalinylen oder der nach dem Weglassen der zwei Aminogruppen des Isophorondiamins erhaltene Rest.

Wenn R einen aromatischen Rest bedeutet, so handelt es sich vorzugsweise um 1,3- oder 1,4-Phenylen oder Naphthylen, die jeweils, falls gewünscht, z.B. auch durch eine oder mehrere $C_1$-$C_4$-Alkylgruppen, wie Methyl, Ethyl oder Propyl, die entsprechenden Alkoxy-, Alkoxycarbonyl oder Alkanoyloxygruppen, oder durch Halogenatome, insbesondere Chlor oder Brom, oder Nitrogruppen substituiert sein können. Bevorzugt sind die genannten Gruppen unsubstituiert oder durch eine Methyl- oder Methoxygruppe substituiert. 1,3- und 1,4-Phenylengruppen sind als aromatische Reste besonders bevorzugt.

Die oben erwähnten aliphatischen und cycloaliphatischen Reste sowie die heterocyclischen Reste R können ebenfalls die bei den aromatischen Resten R aufgeführten Substituenten enthalten.

Als heterocylische Reste R eignen sich insbesondere einen oder zwei O, S oder N Atome enthaltende, gesättigte oder ungesättigte fünf-oder sechsgliedrige Heterocyclen, wie z.B. zweiwertige Reste des Furans, Pyrans, Pyridins, Pyrrols, Imidazols, Thiophens usw.

Besonders bevorzugte Verbindungen der Formel I sind solche, worin R ein $C_2$-$C_{10}$-aliphatischer, ein $C_5$-$C_6$-cycloaliphatischer oder ein $C_6$-$C_{10}$-aromatischer Rest ist oder für eine Gruppe der Formel II steht, worin T Methylen oder Isopropyliden bedeutet.

Wenn R für eine Gruppe der Formel II steht, ist diese bevorzugt in 4,4'-Stellung gebunden.

$R^1$ und $R^2$ können unabhängig voneinander eine verzweigte oder vorzugsweise geradkettige Alkylgruppe mit 1-12, vorzugsweise 1-6 und insbesondere 1 oder 2 C-Atomen sein. Beispiele für geeignete Alkylgruppen sind Dodecyl, Decyl, Octyl, Heptyl, Butyl, Propyl und insbesondere Ethyl oder Methyl.

Wenn $R^1$ und/oder $R^2$ einen Cycloalkylrest bedeuten, so handelt es sich vorzugseise um Cyclopentyl oder Cylcohexyl, die jeweils durch eine oder mehrere $C_1$-$C_4$-Alkylgruppen substituiert sein können. Bevorzugt sind die genannten Gruppen unsubstituiert.

Wenn $R^1$ und/oder $R^2$ einen Arylrest bedeuten, so handelt es sich vorzugsweise um Phenyl, Tolyl, Methoxyphenyl oder Naphthyl. Als $C_7$-$C_{12}$-Aralkyl kommen z.B. Benzyl oder Naphthylmethyl in Frage.

Wenn $R^1$ und/oder $R^2$ einen $C_4$-$C_8$-heterocyclischen bedeuten, kommen z.B. die als mögliche Gruppen R genannten Heterocyclen, diesmal als einwertige Reste, in Frage.

Bevorzugt werden Cyanoguanidine, der Formel I, worin $R^1$ und $R^2$ unabhängig voneinander $C_1$-$C_6$-Alkyl, $C_5$-$C_6$-Cycloalkyl, $C_6$-$C_{10}$-Aryl oder Benzyl bedeuten.

Besonders bevorzugt sind erfindungsgemässe Verbindungen, worin R Phenylen, Methylphenylen, den Rest

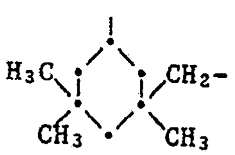

oder eine Gruppe der Formel II, worin T Methylen ist, bedeutet und $R^1$ und $R^2$ unabhängig voneinander Phenyl, Tolyl, Methoxyphenyl, Naphthyl oder Cyclohexyl bedeuten.

Im allgemeinen werden diejenigen oligomeren Cyanoguanidine bevorzugt, bei denen alle drei Reste R, $R^1$ und $R^2$ Gruppen der gleichen Art sind, zum Beispiel Verbindungen, worin diese Gruppen jeweils ein cycloaliphatischer oder jeweils ein aromatischer Rest sind.

Besonders bevorzugt werden Cyanoguanidine der Formel I, worin R, $R^1$ und $R^2$ aromatische Reste sind.

Am meisten bevorzugt sind Verbindungen der Formel I, worin R Methylphenylen oder eine Gruppe der Formel II mit T gleich Methylen ist, und $R^1$ und $R^2$ jeweils Phenyl bedeuten.

Die erfindungsgemässen Cyanoguanidine und Cyanoguanidingemische eignen sich als latente Härter für Epoxidharze. Gegenstand der Erfindung sind daher auch härtbare Stoffgemische enthaltend

(a) ein Epoxidharz und

(b) eine oligomeres Cyanoguanidin der Formel I oder ein erfindungsgemässes Cyanoguanidingemisch als Härter.

Als Epoxidharze (a) kommen alle diejenigen in Betracht, die mit den erfindungsgemässen Cyanoguanidinen ausgehärtet werden können. Insbesondere seien genannt:

Alicyclische Polyepoxide, wie Epoxyethyl-3,4-epoxycyclohexan (Vinylcyclohexendiepoxid), Limonendiepoxid, Dicyclopentadiendiepoxid, Bis(3,4-epoxycyclohexylmethyl)adipat, 3′,4′-Epoxycyclohexylmethyl-3,4-epoxycyclohexancarboxylat, 3′,4′-Epoxy-6′-methylcyclohexylmethyl-3,4-epoxy-6-methylcyclohexancarboxylat, 3-(3′,4′-Epoxycyclohexyl)-2,4-dioxaspiro[5,5]-8,9-epoxyundecan, 3-Glycidyloxyethoxyethyl-2,4-dioxaspiro[5,5]-8,9-epoxyundecan.

Di- oder Polyglycidylether von mehrwertigen Alkoholen, wie 1,4-Butandiol oder Polyalkylenglykolen, wie Polypropylenglykole, Di-oder Polyglycidylether von cycloaliphatischen Polyolen, wie 2,2-Bis(4-hydroxycyclohexyl)-propan, Di- oder Polyglycidylether von mehrwertigen Phenolen, wie Resorcin, Bis(p-hydroxyphenyl)methan (Bisphenol F), 2,2-Bis(p-hydroxyphenyl)propan (Bisphenol A), 2,2-Bis-(4′-hydroxy-3′,5′-dibromphenyl)propan, 1,1,2,2-Tetrakis(p-hydroxyphenyl)ethan, oder unter saueren Bedingungen erhaltene Kondensationsprodukte von Phenolen mit Formaldehyd, wie Phenol-Novolake und Kresol-Novolake ; ferner Di- oder Poly(β-methylglycidyl)ether der oben angeführten Polyalkohole und Polyphenole.

Polyglycidylester und Poly(β-methylglycidyl)ester von mehrwertigen Carbonsäuren, wie Phthalsäure, Terephthalsäure, Tetrahydrophthalsäure und Hexahydrophthalsäure.

N-Glycidylderivate von Aminen, Amiden und heterocyclischen Stickstoffbasen, wie N,N-Diglycidylanilin, N,N-Diglycidyltoluidin, N,N,N′,N′-Tetraglycidyl-bis(p-aminophenyl)methan, Triglycidylisocyanurat, N,N′-Diglycidylethylenharnstoff, N,N′-Diglycidyl-5,5-dimethylhydantoin, N,N′-Diglycidyl-5-isopropyl-hydantoin, N,N′-Diglycidyl-5,5-dimethyl-6-isopropylo-5,6-dihydrouracil.

Gewünschtenfalls kann man den härtbaren Gemischen zur Herabsetzung der Viskosität aktive Verdünner, wie z.B. Styroloxid, Butylglycidylether, 2,2,4-Trimethylpentylglycidylether, Phenylglycidylether, Kresylglycidylether, Glycidylester von synthetischen, hochverzweigten, in der Hauptsache tertiären aliphatischen Monocarbonsäuren zusetzen.

Man kann bei Härtung ausserdem Härtungsbeschleuniger einsetzen ; solche Beschleuniger sind z.B. tertiäre Amine, deren Salze oder quaternäre Ammoniumverbindungen, z.B. Benzyldimethylamin, 2,4,6-Tris(dimethylaminomethyl)phenol, 1-Methylimidazol, 2-Ethyl-4-methylimidazol, N-Acylimidazole, wie z.B. die in den US 4,436,892 und US 4,587,311 beschriebenen Verbindungen, 4-Aminopyridin, Tripentylammoniumphenolat ; oder Alkalimetallalkoholate, wie z.B. Natriumhexantriolat.

Bevorzugt werden erfindungsgemässe härtbare Stoffgemische, welche zusätzlich zu den Komponenten (a) und (b) noch (c) einen Härtungsbeschleuniger, vorzugsweise ein Imidazol-Derivat, enthalten.

Bei den in den erfindungsgemässen härtbaren Stoffgemischen eingesetzten Komponenten (a), (b) und (c) kann es sich jeweils um Einzelverbindungen oder um Gemische handeln.

Die erfindungsgemässen härtbaren Stoffgemische enthalten vorzugsweise 5-25 Gew.%, insbesondere 10-15 Gew.% der Komponente (b) und gegebenenfalls 0,05 -5 Gew.%, vorzugsweise 0,1-1 Gew.%, des Beschleunigers (c), bezogen auf die Menge von (a) + (b).

Ein weiterer Gegenstand der Erfindung ist die Verwendung der erfindungsgemässen härtbaren Stoffgemische zur Herstellung von vernetzten Produkten.

Die Härtung der erfindungsgemässen Mischungen wird zweckmässig im Temperaturintervall von 100°C bis 300°C, bevorzugt von 120-250°C, durchgeführt. Man kann die Härtung in bekannter Weise auch zwei-oder mehrstufig durchführen, wobei die erste Härtungsstufe beiniedriger Temperatur und die Nachhärtung bei höherer Temperatur durchgeführt wird.

Die Härtung kann gewünschtenfalls auch derart in 2 Stufen erfolgen, dass die Härtungsreaktion zunächst vorzeitig abgebrochen bzw. die erste Stufe bei wenig erhöhter Temperatur durchgeführt wird, wobei ein noch schmelzbares und/oder lösliches, härtbares Vorkondensat (sogenannte "B-Stufe") aus der Epoxid-Komponente (a) und dem Härter (b) erhalten wird. Ein derartiges Vorkondensat kann z.B. zur Herstellung von "Prepregs", Pressmassen oder Sinterpulvern dienen.

Der Ausdruck "Harten", wie er hier gebraucht wird, bedeutet die Umwandlung der löslichen, entweder flüssigen oder schmelzbaren Polyepoxide in feste, unlösliche und unschmelzbare, dreidimensional vernetzte Produkte bzw. Werkstoffe, und zwar in der Regel unter gleichzeitiger Formgebung zu Formkörpern, wie Giesskörpern, Presskörpern und Schichtstoffen, zu Imprägnierungen, Beschichtungen, Lackfilmen oder Verklebungen.

Die erfindungsgemässen Stoffgemische eignen sich insbesondere als Laminierharze für die Herstellung von Prepregs und von faserverstärkten Verbundstoffen.

Die nachfolgenden Beispiele erläutern die Erfindung näher.

Herstellungsbeispiele

Beispiel 1 :

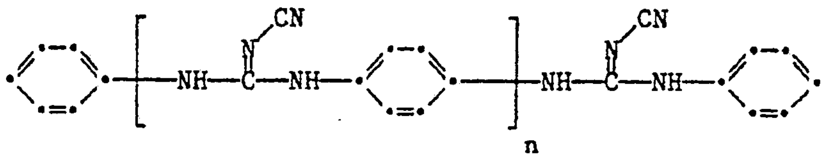

Einen 250 ml Dreihalsrundkolben, versehen mit Rührer, Thermometer, Rückflusskühler sowie Trockenrohr und Blasenzähler beschickt man mit :
40,0 g (336 mMol) Phenylisocyanat
10,0 g (62,5 mMol) 1,4-Phenylendiisocyanat
0,5 g (2,6 mMol) 3-Methyl-1-phenyl-1-phospha-3-cyclopenten-1-oxid und
100 ml Toluol.

Diese Lösung rührt man 2 h unter Rückfluss. Dabei entwickelt sich $CO_2$. Danach entfernt man das Lösungsmittel am Rotationsverdampfer. Zum Rückstand (gelbliches Oel) gibt man :
100 ml Isopropanol
33,6 g (800 mMol) Cyanamid
2,0 ml Triethylamin.

Das Reaktionsgemisch wird während 2 h bei 70-80°C gerührt. Danach kühlt man mit einen Eisbad auf ca. 10°C und filtriert den Niederschlag ab. Nach dem Waschen mit Isopropanol und Trocknen im Vakuum verbleiben 41,0 g weisses Pulver, mit Erweichungspunkt 109-118°C.

Beispiel 2 :

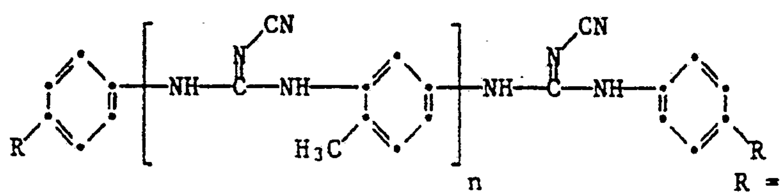

R = H oder $OCH_3$

Einen 1 l-Dreihalsrundkolben, versehen mit Rührer, Thermometer, Rückflusskühler sowie Trockenrohr und Blasenzähler beschickt man mit :
85,0 g (714 mMol) Phenylisocyanat
85,0 g (570 mMol) p-Methoxy-phenylisocyanat
42,5 g (244 mMol) 4-Methyl-m-phenylendiisocyant
2,0 g (10,4 mMol) 3-Methyl-1-phenyl-1-phospha-3-cyclopenten-1-oxid und
400 ml Toluol.

Diese Lösung rührt man 2 h unter Rückfluss. Dabei entwickelt sich $CO_2$. Danach entfernt man das Lösungsmittel am Rotationsverdampfer. Zum Rückstand (gelbliches Oel) gibt man :
400 ml Isopropanol
128 g (3050 mMol) Cyanamid
8 ml Triethylamin.

Das Reaktionsgemisch wird während 2 h bei 70-80°C gerührt. Danach kühlt man mit einem Eisbad auf ca. 10°C und filtriert den Niederschlag ab. Nach dem Waschen mit Isopropanol und Trocknen im Vakuum verbleiben 219 g farbloses Pulver, mit Erweichungspunkt 110-125°C.

Beispiel 3 :

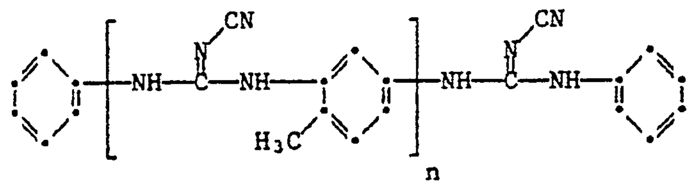

Einen 2 l-Dreihalsrundkolben, versehen mit Rührer, Thermometer, Rückflusskühler sowie Trockenrohr und Blasenzähler beschickt man mit :
240,0 g (2,01 Mol) Phenylisocyanat
160,0 g (0,919 Mol) 4-Methyl-m-phenylen-diisocyanat
2,0 g (0,001 Mol) 3-Methyl-1-phenyl-1-phospha-3-cyclopenten-1-oxid und
900 ml Toluol.

Diese Lösung behandelt man analog Beispiel 1. Zum Rückstand gibt man
1200 ml Isopropanol
246,0 g (5,86 Mol) Cyanamid
15,0 g Triethylamin
und verfährt analog Beispiel 1. Es verbleiben 458 g gelbliches Pulver mit Erweichungspunkt 175-180°C.
Elementaranalyse : (%) C 53,6 ; H 5,6 ; N 37,5

Beispiel 4 :

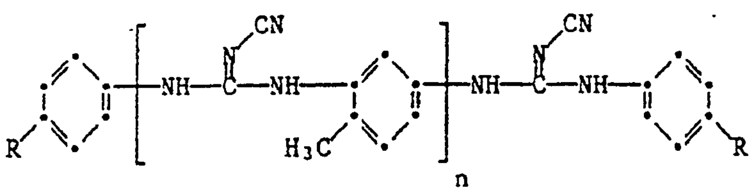

R = H oder $CH_3$

Einen 250 ml Dreihalsrundkolben, versehen mit Rührer, Thermometer, Rückflusskühler sowie Trockenrohr und Blasenzähler beschickt man mit :
15,0 g (126 mMol) Phenylisocyanat
15,0 g (113 mMol) p-Tolylisocyanat
20,0 g (115 mMol) 4-Methyl-m-phenylendiisocyanat
0,5 g (2,6 mMol) 3-Methyl-1-phenyl-1-phospha-3-cyclopenten-1-oxid
100 ml Toluol.

Diese Lösung behandelt man analog Beispiel 1. Zum Rückstand gibt man
100 ml Isopropanol
38,0 g (905 mMol) Cyanamid und
2 ml Triethylamin.

Das Reaktionsgemisch wird während 2 h bei 70-80°C gerührt. Danach kühlt man mit einem Eisbad auf ca. 10°C und filtriert den Niederschlag ab. Nach dem Waschen mit Isopropanol und Trocknen im Vakuum verbleiben 47,4 g farbloses Pulver mit Erweichungsbereich 180-185°C.

Beispiel 5 :

In diesem Versuch werden die gleichen Edukte wie in Beispiel 3 umgesetzt. Hingegen wurde das Endprodukt anders isoliert. Einen 250 ml Dreihalsrundkolben, versehen mit Rührer, Thermometer, Rückflusskühler sowie Trockenrohr und Blasenzähler beschickt man mit :
30,0 (252 mMol) Phenylisocyanat
20,0 g (115 mMol) 4-Methyl-m-phenylendiisocyanat
0,5 (2,6 mMol) 3-Methyl-1-phenyl-1-phospha-3-cyclopenten-1-oxid und
100 ml Toluol.

Diese Lösung behandelt man analog Beispiel 1. Das dabei erhaltene Carbodiimidgemisch hat ein durch Gelpermeationschromatographie (THF, Polystyrol-Standard) ermitteltes Molekulargewicht von $\overline{M}_n$ = 318 und

$\overline{M}_w = 469$.

Zu diesem Gemisch gibt man :
120 ml Isopropanol
80 ml Tetrahydrofuran
2 ml Triethylamin und
19 g (452 mMol) Cyanamid. Das Reaktionsgemisch wird während 2 h bei 70-80°C gerührt. Danach giesst man auf 500 ml Wasser und filtriert den farblosen Niederschlag ab. Nach dem Trocknen im Vakuum verbleiben 48,0 g schwach gelbliches Pulver. Erweichungsbereich 147-164°C.
Elementaranalyse : gefunden (%) : C 65,55 H 5,09 N 28,22.

Beispiel 6 :

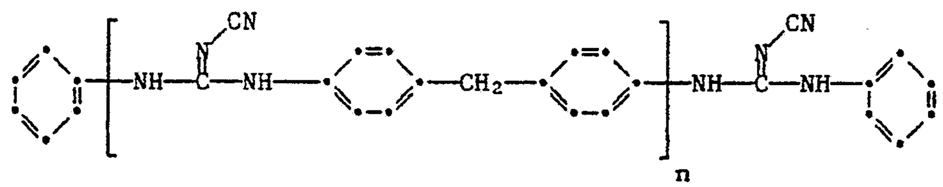

Einen 250 ml Dreihalsrundkolben, versehen mit Rührer, Thermometer, Rückflusskühler sowie Trockenrohr und Blasenzähler beschickt man mit :
35,0 g (294 mMol) Phenylisocyanat
15,0 g (60 mMol) Diphenylmethan-4,4'-diisocyanat
0,1 g (0,52 mMol) 3-Methyl-1-phenyl-1-phospha-3-cyclopenten-1-oxid
100 ml Toluol.

Diese Lösung behandelt man analog Beispiel 1. Diesen Rückstand tropft man in 1 h Stunde bei Raumtemperatur zu einer Lösung bestehend aus :
100 ml 1-Methoxy-2-propanol
15,0 g (357 mMol) Cyanamid
0,5 ml Triethylamin.

Dabei steigt die Temperatur auf 45°C. Danach rührt man noch 4 h bei Raumtemperatur und giesst die Reaktionslösung auf 300 ml Wasser und filtriert den Niederschlag ab. Nach dem Waschen mit Wasser und Trocknen im Vakuum verbleiben 48,1 g farbloses Pulver mit einem Erweichungsbereich von 162-169°C.

Anwendungsbeispiele

$A_1$) 10 Teile des gemäss Beispiel 3 hergestellten Härters werden mit 90 Teilen Bisphenol-A-diglycidylether (Epoxidgehalt 5,4 Aequivalente/kg) vermischt. Beim Erwärmen dieser Mischung setzt bei 90°C eine exotherme Reaktion ein. Dabei werden zwischen 90 und 200°C 290 J/g Wärme freigesetzt. Es zeigen sich zwei Exothermiemaxima, nämlich bei 140 und 163°C. Diese Mischung hat folgende Gelierzeiten :
110 s bei 200°C
270 s bei 180°C
630 s bei 160°C.
Die Mischung härtet man 4 h bei 180°C. Dabei entsteht ein klarer Giesskörper mit einer Glasumwandlungstemperatur (DSC) von 164°C.

$A_2$) 10 Teile des gemäss Beispiel 2 hergestellten Härters werden mit 90 Teilen Bisphenol-A-diglycidylether (Epoxidgehalt 5,4 Aequivalente/kg) vermischt. Beim Erwärmen dieser Mischung setzt bei 105°C eine exotherme Reaktion ein. Dabei werden zwischen 105 und 210°C 320 J/g Wärme freigesetzt. Es zeigen sich zwei Energiemaxima, nämlich bei 152 und 178°C. Diese Mischung hat folgende Gelierzeiten :
115 s bei 200°C
350 s bei 180°C
1140 s bei 160°C.
Die Mischung härtet man 4 h bei 180°C. Dabei entsteht ein klarer gelblicher Giesskörper mit einer Glasumwandlungstemperatur von 167°C.

$A_3$) 10 Teile des gemäss Beispiel 4 hergestellten Härters werden mit 90 Teilen Bisphenol-A-diglycidylether (Epoxidgehalt 5,4 Aequivalente/kg) gemischt. Beim Erwärmen dieser Mischung setzt bei 90°C eine exotherme Reaktion ein. Dabei werden zwischen 90 und 210°C 370 J/g Wärme freigesetzt. Es zeigen sich zwei Exothermiemaxima, nämlich bei 145 und 171°C. Diese Mischung hat folgende Gelierzeiten :

135 s bei 200°C
270 s bei 180°C
780 s bei 160°C.
Die Mischung härtet man 4 h bei 180°C. Dabei entsteht ein klarer gelblicher Giesskörper mit einer Glasumwandlungstemperatur (DSC) von 158°C.

$A_4$) 15 Teile des gemäss Beispiel 5 hergestellten Härters werden mit 85 Teilen Bisphenol-A-diglycidylether (Epoxidgehalt 5,4 Aequivalente/kg) gemischt. Diese Mischung geliert bei 170°C in 900 s. Fügt man zu dieser Mischung 0,2 Teile 2-Ethylimidazol, so reduziert sich die Gelierzeit auf 120 s. Härtet man die Mischungen 4 h bei 180°C, so entstehen gelbliche klare Giesskörper mit einer Glasumwandlungstemperatur von 165°C.

$A_5$) 10 Teile des gemäss Beispiel 6 hergestellten Härters werden mit 90 Teilen Bisphenol-A-diglycidylether (Epoxidgehalt 5,4 Aequivalente/kg) und 0,5 Teilen 2-Ethylimidazol gemischt. Beim Erwärmen dieser Mischung setzt bei 90°C eine exotherme Reaktion ein. Härtet man diese Mischung 4 h bei 180°C so entsteht ein klarer gelblicher Giesskörper mit einer Glasumwandlungstemperatur (DSC) von 154°C.

$A_6$) 10 Teile des gemäss Beispiel 5 hergestellten Härters werden mit 90 Teilen Bisphenol-A-diglycidylether (Epoxidgehalt 5,4 Aequivalente/kg) und 0,5 Teilen 2-Ethylimidazol gemischt und jeweils eine Stunde bei 150, 160 und 190°C gehärtet. Dabei erhält man einen klaren gelben Giesskörper mit folgenden Eigenschaften :

Glasumwandlungstemperatur (DSC): 150°C
Biegefestigkeit (ISO 178): 119 $N/mm^2$
Randfaserdehnung (ISO 178): 5,9 %
Schlagzähigkeit (ISO/R 179): 22,9 $kJ/m^2$
Kaltwasseraufnahme (4 Tage): 0,30 %
Kochwasseraufnahme (1 h): 0,30 %
Gewichtsverlust (-5 %): 325°C
(-10 %): 360°C

## Patentansprüche

### Patentansprüche für folgende Vertragsstaaten : AT, BE, CH, DE, FR, GB, IT, LI, NL, SE

1. Oligomere Cyanoguanidine der Formel I

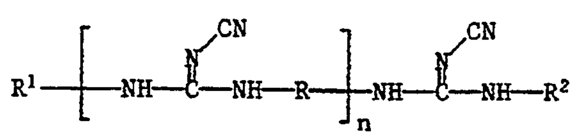
(I),

worin R ein zweiwertiger $C_2$-$C_{20}$-aliphatischer, ein- oder mehrkeniger $C_5$-$C_{20}$-cycloaliphatischer, $C_6$-$C_{20}$-aromatischer oder $C_4$-$C_{20}$-heterocyclischer Rest ist oder für eine Gruppe der Formel II steht

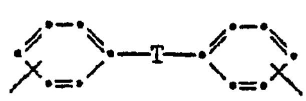
(II),

worin T Methylen, Isopropyliden, CO, O, S oder $SO_2$ bedeutet, $R^1$ und $R^2$ unabhängig voneinander einen $C_1$-$C_{12}$-Alkyl-, $C_5$-$C_{10}$-Cycloalkyl-, $C_6$-$C_{10}$-Aryl-, $C_7$-$C_{12}$-Aralkyl- oder einen $C_3$-$C_8$-heterocyclischen Rest bedeuten und n eine ganze Zahl von 1-20 ist, wobei die Reste R, $R^1$ und $R^2$ unsubstituiert sind oder $C_1$-$C_4$-Alkyl, $C_1$-$C_4$-Alkoxy-, Nitro-, Halogen-,

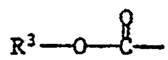 oder 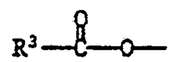

Substituenten enthalten und $R^3$ Phenyl oder $C_1$-$C_4$-Alkyl bedeutet, wobei die zwei folgenden Verbindungen 1,6-Hexan-bis-(3-cyano-2-isobutylguanidin) und 1,8-Bis-[N'-(3-(2-thiazolyl)-propyl)-N''-cyanoguanidino]-octan ausgenommen sind.

2. Cyanoguanidine nach Anspruch 1, worin n eine ganze Zahl von 1-10 ist.

3. Cyanoguanidine nach Anspruch 1, worin R ein $C_2$-$C_{10}$-aliphatischer, ein $C_5$-$C_6$-cycloaliphatischer oder ein $C_6$-$C_{10}$-aromatischer Rest ist oder für eine Gruppe der Formel II nach Anspruch 1 steht, worin T Methylen oder Isopropyliden bedeutet.

4. Cyanoguanidine nach Anspruch 1, worin $R^1$ und $R^2$ unabhängig voneinander $C_1$-$C_6$-Alkyl, $C_5$-$C_6$-Cycloalkyl, $C_6$-$C_{10}$-Aryl oder Benzyl bedeuten.

5. Cyanoguanidine nach Anspruch 1, worin R Phenylen, Methylphenylen, den Rest

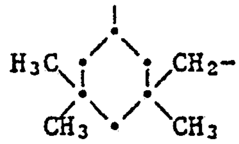

oder eine Gruppe der Formel II, worin T Methylen ist, bedeutet, und $R^1$ und $R^2$ unabhängig voneinander Phenyl, Tolyl, Methoxyphenyl, Naphthyl oder Cyclohexyl bedeuten.

6. Cyanoguanidine nach Anspruch 1, worin R, $R^1$ und $R^2$ aromatische Reste sind.

7. Cyanoguanidine nach Anspruch 1, worin R Methylphenylen oder eine Gruppe der Formel II mit T gleich Methylen ist, und $R^1$ und $R^2$ jeweils Phenyl bedeuten.

8. Verfahren zur Herstellung von Cyanoguanidinen der Formel I nach Anspruch 1 durch Erhitzen eines Gemisches enthaltend ein Monoisocyanat $R^1$-NCO und $R^2$-NCO und ein Diisocyanat OCN-R-NCO in Gegenwart eines Katalysators zu einem oligomeren Carbodiimdid der Formel III

$$R^1-\left[-N{=}C{=}N-R-\right]_n-N{=}C{=}N-R^2 \qquad \text{(III)},$$

und anschliessende Umsetzung des Carbodiimids der Formel III mit Cyanamid, wobei die Symbole $R^1$, $R^2$, R und n die im Anspruch 1 angegebene Bedeutung haben.

9. Cyanoguanidingemisch erhältlich durch Erhitzen eines Gemisches enthaltend ein Monoisocyanat $R^1$-NCO und $R^2$-NCO und ein Diisocyanat OCN-R-NCO in Gegenwart eines Katalysators zu einem Carbodiimid der Formel III*

$$R^1-\left[-N{=}C{=}N-R-\right]_{n^*}-N{=}C{=}N-R^2 \qquad \text{(III*)},$$

und anschliessende Ursetzung des Carbodiimids der Formel III* mit Cyanamid, wobei die Symbole $R^1$, $R^2$ und R die im Anspruch 1 angegebene Bedeutung haben, und n* Null oder eine ganze Zahl von 1-20 ist.

10. Härtbares Stoffgemisch enthaltend

(a) ein Epoxidharz und

(b) ein oligomeres Cyanoguanidin der Formel I nach Anspruch 1 oder ein Cyanoguanidingemisch nach Anspruch 9 als Härter.

11. Stoffgemisch nach Anspruch 10, enthaltend zusätzlich (c) einen Härtungsbeschleuniger.

12. Stoffgemisch nach den Ansprüchen 10 oder 11, enthaltend 5-25 Gew.% der Komponente (b) und gegebenenfalls 0,05 -5 Gew.% des Beschleunigers (c), bezogen auf die Menge von (a) + (b).

13. Verwendung des Stoffgemisches nach Anspruch 10 zur Herstellung von vernetzten Produkten.

**Patentansprüche für folgenden Vertragsstaat : ES**

1. Verfahren zur Herstellung von oligomeren Cyanoguanidinen der Formel I

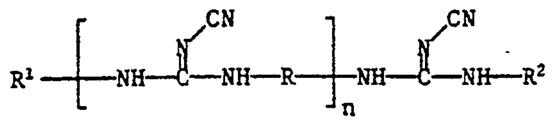

(I),

worin R ein zweiwertiger $C_2$-$C_{20}$-aliphatischer, ein- oder mehrkerniger $C_5$-$C_{20}$-cycloaliphatischer, $C_6$-$C_{20}$-aromatischer oder $C_4$-$C_{20}$-heterocyclischer Rest ist oder für eine Gruppe der Formel II steht

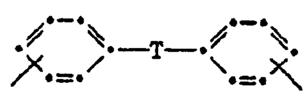

(II),

worin T Methylen, Isopropyliden, CO, O, S oder $SO_2$ bedeutet, $R^1$ und $R^2$ unabhängig voneinander einen $C_1$-$C_{12}$-Alkyl-, $C_5$-$C_{10}$-Cycloalkyl-, $C_6$-$C_{10}$-Aryl-, $C_7$-$C_{12}$-Aralkyl- oder einen $C_3$-$C_8$-heterocyclischen Rest bedeuten und n eine ganze Zahl von 1-20 ist, wobei die Reste R, $R^1$ und $R^2$ unsubstituiert sind oder $C_1$-$C_4$-Alkyl, $C_1$-$C_4$-Alkoxy-, Nitro-, Halogen-,

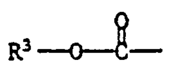 oder 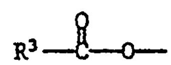

Substituenten enthalten und $R^3$ Phenyl oder $C_1$-$C_4$-Alkyl bedeutet, durch Erhitzen eines Gemisches enthaltend ein Monoisocyanat $R^1$-NCO und/oder $R^2$-NCO und ein Diisocyanat OCN-R-NCO in Gegenwart eines Katalysators zu einem oligomeren Carbodiimdid der Formel III

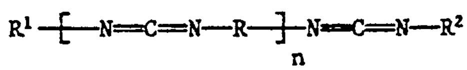

(III),

und anschliessende Umsetzung des Carbodiimids der Formel III mit Cyanamid, wobei die Symbole $R^1$, $R^2$, R und n die unter Formel I angegebene Bedeutung haben.

2. Verfahren nach Anspruch 1, worin n eine ganze Zahl von 1-10 ist.

3. Verfahren nach Anspruch 1, worin R ein $C_2$-$C_{10}$-aliphatischer, ein $C_5$-$C_6$-cycloaliphatischer oder ein $C_6$-$C_{10}$-aromatischer Rest ist oder für eine Gruppe der Formel II nach Anspruch 1 steht, worin T Methylen oder Isopropyliden bedeutet.

4. Verfahren nach Anspruch 1, worin $R^1$ und $R^2$ unabhängig voneinander $C_1$-$C_6$-Alkyl, $C_5$-$C_6$-Cycloalkyl, $C_6$-$C_{10}$-Aryl oder Benzyl bedeuten.

5. Verfahren nach Anspruch 1, worin R Phenylen, Methylphenylen, den Rest

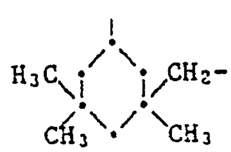

oder eine Gruppe der Formel II, worin T Methylen ist, bedeutet, und $R^1$ und $R^2$ unabhängig voneinander Phenyl, Tolyl, Methoxyphenyl, Naphthyl oder Cyclohexyl bedeuten.

6. Verfahren nach Anspruch 1, worin R, $R^1$ und $R^2$ aromatische Reste sind.

7. Verfahren nach Anspruch 1, worin R Methylphenylen oder eine Gruppe der Formel II mit T gleich Methylen ist, und $R^1$ und $R^2$ jeweils Phenyl bedeuten.

8. Cyanoguanidingemisch erhältlich durch Erhitzen eines Gemisches enthaltend ein Monoisocyanat $R^1$-

NCO und/ $R^2$-NCO und ein Diisocyanat OCN-R-NCO in Gegenwart eines Katalysators zu einem Carbodiimid der Formel III*

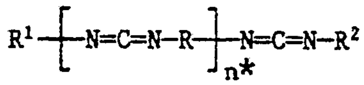

(III*),

und anschliessende Umsetzung des Carbodiimids der Formel III* mit Cyanamid, wobei die Symbole $R^1$, $R^2$ und R die im Anspruch 1 angegebene Bedeutung haben, und n* Null oder eine ganze Zahl von 1-20 ist.

9. Härtbares Stoffgemisch enthaltend

(a) ein Epoxidharz und

(b) eine oligomeres Cyanoguanidin der Formel I nach Anspruch 1 oder ein Cyanoguanidingemisch nach Anspruch 8 als Härter.

10. Stoffgemisch nach Anspruch 9, enthaltend zusätzlich (c) einen Härtungsgbeschleuniger.

11. Stoffgemisch nach den Ansprüchen 9 oder 10, enthaltend 5-25 Gew.% der Komponente (b) und gegebenenfalls 0,05 -5 Gew.% des Beschleunigers (c), bezogen auf die Menge von (a) + (b).

12. Verwendung des Stoffgemisches nach Anspruch 9 zur Herstellung von vernetzten Produkten.

## Claims

**Claims for the following Contracting States : AT, BE, CH, DE, FR, GB, IT, LI, NL, SE**

1. An oligomeric cyanoguanidine of formula I

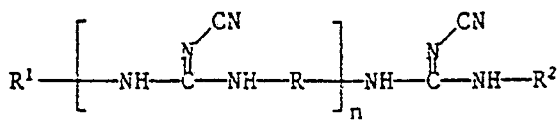

(I),

in which R is a divalent $C_2$-$C_{20}$aliphatic, mono- or polynuclear $C_5$-$C_{20}$-cycloaliphatic, $C_6$-$C_{20}$aromatic or $C_4$-$C_{20}$heterocyclic radical or a group of formula II

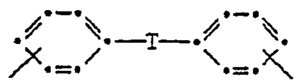

(II),

in which T is methylene, isopropylidene, CO, O, S or $SO_2$, $R^1$ and $R^2$ are each independently of the other a $C_1$-$C_{12}$alkyl, $C_5$-$C_{10}$cycloalkyl, $C_6$-$C_{10}$aryl, $C_7$-$C_{12}$aralkyl or $C_3$-$C_8$heterocyclic radical, and n is an integer from 1 to 20, where the radicals R, $R^1$ and $R^2$ are unsubstituted or are substituted by $C_1$-$C_4$alkyl, $C_1$-$C_4$alkoxy, nitro, halogen, $R^3$-O-C— or $R_3$-C-O— and $R^3$ is phenyl or $C_1$-$C_4$alkyl, with the exception of the two following compounds, 1,6-hexane-bis(3-cyano-2-isobutylguanidine) and 1,8-bis[N'(3-(2-thiazolyl)propyl)-N"-cyanoguanidino]octane.

2. A cyanoguanidine according to claim 1, in which n is an integer from 1 to 10.

3. A cyanoguanidine according to claim 1, in which R is a $C_2$-$C_{10}$aliphatic, $C_5$-$C_6$cycloaliphatic or $C_6$-$C_{10}$aromatic radical or is a group of formula II according to claim 1, in which T is methylene or isopropylidene.

4. A cyanoguanidine according to claim 1, in which $R^1$ and $R^2$ are each independently of the other $C_1$-$C_6$alkyl, $C_5$-$C_6$cycloalkyl, $C_6$-$C_{10}$aryl or benzyl.

5. A cyanoguanidine according to claim 1, in which R is phenylene, methylphenylene, the radical

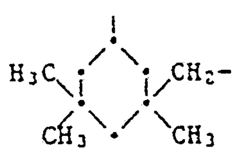

or a group of formula II, in which T is methylene, and $R^1$ and $R^2$ are each independently of the other phenyl, tolyl, methoxyphenyl, naphthyl or cyclohexyl.

6. A cyanoguanidine according to claim 1, in which R, $R^1$ and $R^2$ are aromatic radicals.

7. A cyanoguanidine according to claim 1, in which R is methylphenylene or a group of formula II, in which T is methylene, $R^1$ and $R^2$ are each phenyl.

8. A process for the preparation of a cyanoguanidine of formula I as claimed in claim 1, which comprises heating a mixture containing a monoisocyanate $R^1$-NCO and $R^2$-NCO and a diisocyanate OCN-R-NCO, in the presence of a catalyst, to give an oligomeric carbodiimide of formula III

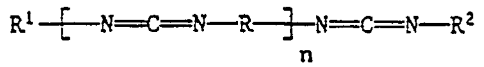
(III),

and subsequently reacting the carbodiimide of formula III with cyanamide, where the symbols $R^1$, $R^2$, R and n are as defined in claim 1.

9. A mixture of cyanoguanidines obtainable by heating a mixture containing a monoisocyanate $R^1$-NCO and $R^2$-NCO and a diisocyanate OCN-R-NCO, in the presence of a catalyst, to give a carbodiimide of formula III*

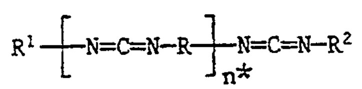
(III*),

and subsequently reacting the carbodiimide of formula III* with cyanamide, where the symbols $R^1$, $R^2$ and R are as defined in claim 1, and n* is zero or an integer from 1 to 20.

10. A curable composition comprising

(a) an epoxy resin, and

(b) an oligomeric cyanoguanidine of formula I according to claim 1, or a mixture of cyanoguanidines according to claim 9, as hardener.

11. A composition according to claim 10, which additionally contains (c) a curing accelerator.

12. A composition according to claim 10 or claim 11, which contains 5-25% by weight of component (b) and, optionally, 0.05-5% by weight of the accelerator (c), based on the amount of (a) + (b).

13. The use of a composition according to claim 10 for the preparation of crosslinked products.

**Claims for the following Contracting States : ES**

1. A process for the preparation of an oligomeric cyanoguanidine of formula I

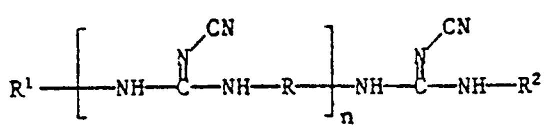
(I),

in which R is a divalent $C_2$-$C_{20}$aliphatic, mono- or polynuclear $C_5$-$C_{20}$cycloaliphatic, $C_6$-$C_{20}$aromatic or $C_4$-$C_{20}$heterocyclic radical or a group of formula II

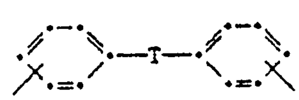
(II),

in which T is methylene, isopropylidene, CO, O, S or $SO_2$, $R^1$ and $R^2$ are each independently of the other a $C_1$-$C_{12}$alkyl, $C_5$-$C_{10}$cycloalkyl, $C_6$-$C_{10}$aryl, $C_7$-$C_{12}$aralkyl or $C_3$-$C_8$heterocyclic radical, and n is an integer from 1 to 20, where the radicals R, $R^1$ and $R^2$ are unsubstituted or are substituted by $C_1$-$C_4$alkyl, $C_1$-$C_4$alkoxy, nitro, halogen, or $R^3$-O-C— or $R^3$-C-O— and $R^3$ is phenyl or $C_1$-$C_4$alkyl which comprises heating a mixture containing a monoisocyanate $R^1$-NCO and $R^2$-NCO and a diisocyanate OCN-R-NCO, in the presence of a catalyst, to give an oligomeric carbodiimide of formula III

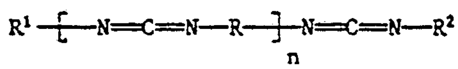

(III),

and subsequently reacting the carbodiimide of formula III with cyanamide, where the symbols $R^1$, $R^2$, R and n are as defined for formula I.

2. A process according to claim 1, in which n is an integer from 1 to 10.

3. A process according to claim 1, in which R is a $C_2$-$C_{10}$aliphatic, a $C_5$-$C_6$cycloaliphatic or a $C_6$-$C_{10}$aromatic radical or is a group of formula II according to claim 1, in which T is methylene or isopropylidene.

4. A process according to claim 1, in which $R^1$ and $R^2$ are each independently of the other $C_1$-$C_6$alkyl, $C_5$-$C_6$cycloalkyl, $C_6$-$C_{10}$aryl or benzyl.

5. A process according to claim 1, in which R is phenylene, methylphenylene, the radical

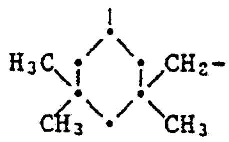

or a group of formula II, in which T is methylene, and $R^1$ and $R^2$ are each independently of the other phenyl, tolyl, methoxyphenyl, naphthyl or cyclohexyl.

6. A process according to claim 1, in which R, $R^1$ and $R^2$ are aromatic radicals.

7. A process according to claim 1, in which R is methylphenylene or a group of formula II, in which T is methylene, and $R^1$ and $R^2$ are each phenyl.

8. A mixture of cyanoguanidines obtainable by heating a mixture containing a monoisocyanate $R^1$-NCO and $R^2$-NCO and a diisocyanate OCN-R-NCO, in the presence of a catalyst, to give a carbodiimide of formula III*

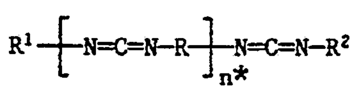

(III*)

and subsequently reacting the carbodiimide of formula III* with cyanamide, where the symbols $R^1$, $R^2$ and R are as defined in claim 1 and n* is zero or an integer from 1 to 20.

9. A curable composition comprising

(a) an epoxy resin, and

(b) an oligomeric cyanoguanidine of formula I according to claim 1, or a mixture of cyanoguanidines as claimed in claim 8, as hardener.

10. A composition according to claim 9, which additionally contains (c) a curing accelerator.

11. A composition according to claim 9 or claim 10, which contains 5-25% by weight of component (b) and, optionally, 0.05-5% by weight of the accelerator (c), based on the amount of (a) + (b).

12. The use of a composition according to claim 9 for the preparation of crosslinked products.

## Revendications

### Revendications pour les Etats contractants suivants : AT, BE, CH, DE, FR, GB, IT, LI, NL, SE

1. Cyanoguanidines oligomères répondant à la formule I

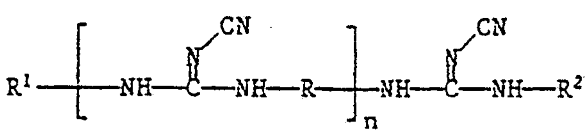

(I)

dans laquelle

R représente un radical bivalent qui est un radical aliphatique en $C_2$-$C_{40}$, un radical cycloaliphatique en $C_5$-$C_{20}$ à un ou à plusieurs noyaux, un radical aromatique en $C_6$-$C_{20}$, un radical hétérocyclique en $C_4$-$C_{20}$ ou un radical de formule II

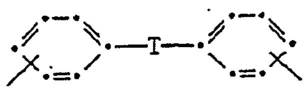

(II)

dans lequel T représente un radical méthylène, isopropylidène, CO, O, S ou $SO_2$,

$R^1$ et $R^2$ représentent chacun, indépendamment l'un de l'autre, un alkyle en $C_1$-$C_{12}$, un cycloalkyle en $C_5$-$C_{10}$, un aryle en $C_6$-$C_{10}$, un aralkyle en $C_7$-$C_{12}$ ou un un radical hétérocyclique en $C_3$-$C_8$, et

n désigne un nombre de 1 à 20, les radicaux R, $R^1$ et $R^2$ étant dépourvus de substituants ou portant un alkyle en $C_1$-$C_4$, en alcoxy en $C_1$-$C_4$, un nitro, un halogène ou un radical ou où le symbole $R^3$ représente un phényle ou un alkyle en $C_1$-$C_4$,

à l'exclusion des deux composés suivants : le bis-(cyano-3 isobutyl-2 guanidino)-1,6 hexane et le bis-[N'-((thiazoyl-2)-3 propyl)-N"-cyano-guanidino]-1,8 octane.

2. Cyanoguanidines selon la revendication 1 dans lesquelles n désigne un nombre entier de 1 à 10.

3. Cyanoguanidines selon la revendication 1, dans lesquelles R représente un radical aliphatique en $C_2$-$C_{10}$, cycloaliphatique en $C_5$-$C_6$ ou aromatique en $C_6$-$C_{10}$ ou représente un radical de formule II selon la revendication 1, dans lequel T représente un radical méthylène ou un isopropylidène.

4. Cyanoguanidines selon la revendication 1, dans lesquelles $R^1$ et $R^2$ représentent chacun, indépendamment l'un de l'autre, un alkyle en $C_1$-$C_6$, un cycloalkyle en $C_5$ ou $C_6$, un aryle en $C_6$-$C_{10}$ ou un benzyle.

5. Cyanoguanidines selon la revendication 1, dans lesquelles R représente un phénylène, un méthylphénylène, le radical :

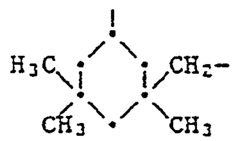

ou un radical de formule II dans lequel T représente un méthylène, et $R^1$ et $R^2$ représentent chacun, indépendamment l'un de l'autre, un radical phényle, tolyle, méthoxyphényle, naphtyle ou cyclohexyle.

6. Cyanoguanidines selon la revendication 1 dans lesquelles R, $R^1$ et $R^2$ représentent des radicaux aromatiques.

7. Cyanoguanidines selon la revendication 1 dans lesquelles R représente un méthylphénylène ou un radical de formule II dans lequel T représente un méthylène, et $R^1$ et $R^2$ représentent chacun un phényle.

8. Procédé pour préparer des cyanoguanidines de formule I selon la revendication 1, procédé selon lequel on chauffe un mélange contenant un mono-isocyanate $R^1$-NCO, un monoisocyanate $R^2$-NCO et un di-isocyanate OCN-R-NCO en présence d'un catalyseur, réaction qui conduit à un carbodiimide oligomère de formule III

$$R^1-\left[-N=C=N-R-\right]_n-N=C=N-R^2 \qquad \text{(III)},$$

puis on fait réagir le carbodiimide de formule III avec le cyanamide, les symboles $R^1$, $R^2$, R et n ayant les significations qui leur ont été données à la revendication 1.

9. Mélange de cyanoguanidines qui peut être obtenu par chauffage d'un mélange contenant un mono-isocyanate $R^1$-NCO, un mono-isocyanate $R^2$-NCO et un di-isocyanate OCN-R-NCO en présence d'un catalyseur, réaction qui conduit à un carbodiimide de formule III*

$$R^1-\left[-N=C=N-R-\right]_{n^*}-N=C=N-R^2 \qquad \text{(III*)},$$

puis réaction du carbodiimide de formule III* avec le cyanamide ; les symboles $R^1$, $R^2$ et R ayant les significations qui leur ont été données à la revendication 1, et n* étant égal à 0 ou désignant un nombre entier de 1 à

20.

10. Mélange durcissable qui contient :

(a) Une résine époxydique et

(b) Une cyanoguanidine oligomère de formule I selon la revendication 1 ou un mélange de cyanoguanidines selon la revendication 9, comme durcisseur.

11. Mélange selon la revendication 10 qui contient en outre (c) un accélérateur de durcissement.

12. Mélange selon l'une des revendications 10 et 11 qui contient 5 à 25% en poids de la composante (b) et, éventuellement, de 0,05 à 5% en poids de l'accélérateur (c), par rapport à la quantité de (a) + (b).

13. Application du mélange selon la revendication 10 à la préparation de produits réticulés durcis.

**Revendications pour l'Etat contractant suivant : ES**

1. Procédé de préparation de cyanoguanidines oligomères à la formule I

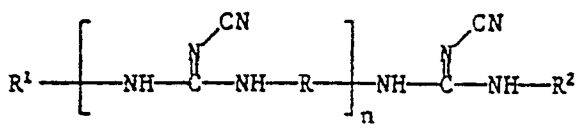

(I)

dans laquelle

R représente un radical bivalent qui est un radical aliphatique en $C_2$-$C_{40}$, un radical cycloaliphatique en $C_5$-$C_{20}$ à un ou à plusieurs noyaux, un radical aromatique en $C_6$-$C_{20}$, un radical hétérocyclique en $C_4$-$C_{20}$ ou un radical de formule II

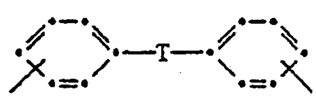

(II)

dans lequel T représente un radical méthylène, isopropylidène, CO, O, S ou $SO_2$,

$R^1$ et $R^2$ représentent chacun, indépendamment l'un de l'autre, un alkyle en $C_1$-$C_{12}$, un cycloalkyle en $C_5$-$C_{10}$, un aryle en $C_6$-$C_{10}$, un aralkyle en $C_7$-$C_{12}$ ou un un radical hétérocyclique en $C_3$-$C_8$, et

n désigne un nombre de 1 à 20, les radicaux R, $R^1$ et $R^2$ étant dépourvus de substituants ou portant un alkyle en $C_1$-$C_4$, en alcoxy en $C_1$-$C_4$, un nitro, un halogène ou un radical

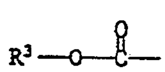 ou 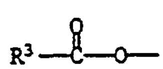

où le symbole $R^3$ représente un phényle ou un alkyle en $C_1$-$C_4$. Procédé selon lequel on chauffe un mélange contenant un mono-isocyanate $R^1$-NCO, un mono-isocyanate $R^2$-NCO et un diisocyanate OCN-R-NCO en présence d'un catalyseur, réaction qui conduit à un carbodiimide oligomère de formule III

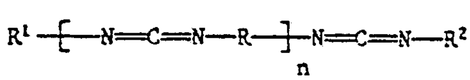

(III),

puis on fait réagir le carbodiimide de formule III avec le cyanamide, les symboles $R^1$, $R^2$, R et n ayant les significations qui leur ont été données à la revendication 1.

2. Procédé selon la revendication 1 dans lequel n désigne un nombre entier de 1 à 10.

3. Procédé selon la revendication 1, dans lequel R représente un radical aliphatique en $C_2$-$C_{10}$, cycloaliphatique en $C_5$-$C_6$ ou aromatique en $C_6$-$C_{10}$ ou représente un radical de formule II selon la revendication 1, dans lequel T représente un radical méthylène ou un isopropylidène.

4. Procédé selon la revendication 1, dans lequel $R^1$ et $R^2$ représentent chacun, indépendamment l'un de l'autre, un alkyle en $C_1$-$C_6$, un cycloalkyle en $C_5$ ou $C_6$, un aryle en $C_6$-$C_{10}$ ou un benzyle.

5. Procédé selon la revendication 1, dans lequel R représente un phénylène, un méthylphénylène, le radi-

cal :

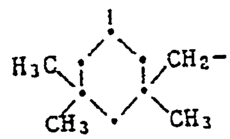

ou un radical de formule II dans lequel T représente un méthylène, et $R^1$ et $R^2$ représentent chacun, indépendamment l'un de l'autre, un radical phényle, tolyle, méthoxyphényle, naphtyle ou cyclohexyle.

6. Procédé selon la revendication 1 dans lequel R, $R^1$ et $R^2$ représentent des radicaux aromatiques.

7. Procédé selon la revendication 1 dans lequel R représente un méthylphénylène ou un radical de formule II dans lequel T représente un méthylène, et $R^1$ et $R^2$ représentent chacun un phényle.

8. Mélange de cyanoguanidines qui peut être obtenu par chauffage d'un mélange contenant un mono-isocyanate $R^1$-NCO, un mono-isocyanate $R^2$-NCO et un di-isocyanate OCN-R-NCO en présence d'un catalyseur, réaction qui conduit à un carbodiimide de formule III*

$$R^1 - \left[ -N{=}C{=}N-R- \right]_{n^*} -N{=}C{=}N-R^2 \qquad \text{(III*)},$$

puis réaction du carbodiimide de formule III* avec le cyanamide ; les symboles $R^1$, $R^2$ et R ayant les significations qui leur ont été données à la revendication 1, et n* étant égal à 0 ou désignant un nombre entier de 1 à 20.

9. Mélange durcissable qui contient :

(a) Une résine époxydique et

(b) Une cyanoguanidine oligomère de formule I selon la revendication 1 ou un mélange de cyanoguanidines selon la revendication 8, comme durcisseur.

10. Mélange selon la revendication 9 qui contient en outre (c) un accélérateur de durcissement.

11. Mélange selon l'une des revendications 9 et 10 qui contient 5 à 25% en poids de la composante (b) et, éventuellement, de 0,05 à 5% en poids de l'accélérateur (c), par rapport à la quantité de (a) + (b).

12. Application du mélange selon la revendication 9 à la préparation de produits réticulés durcis.